(19) Europäisches Patentamt
European Patent Office
Office européen des brevets

(11) Veröffentlichungsnummer: **0 036 835**
**B1**

(12) **EUROPÄISCHE PATENTSCHRIFT**

(45) Veröffentlichungstag der Patentschrift: **28.09.83**

(21) Anmeldenummer: **81810083.6**

(22) Anmeldetag: **09.03.81**

(51) Int. Cl.³: **C 09 B 57/04,** C 07 D 403/12, C 07 D 209/44, C 08 K 5/34, C 09 B 26/02

(54) **Verfahren zur Herstellung von Metallkomplexen von Isoindolinazinen.**

(30) Priorität: **13.03.80 CH 1977/80**

(43) Veröffentlichungstag der Anmeldung: **30.09.81 Patentblatt 81/39**

(45) Bekanntmachung des Hinweises auf die Patenterteilung: **28.09.83 Patentblatt 83/39**

(84) Benannte Vertragsstaaten: **CH DE FR GB IT LI**

(56) Entgegenhaltungen:
**EP-A-0 020 299**
**DE-A-2 504 321**

(73) Patentinhaber: **CIBA-GEIGY AG, Patentabteilung Postfach, CH-4002 Basel (CH)**

(72) Erfinder: **Iqbal, Abul, Dr., Im Schaiengarten 1, CH-4107 Ettingen (CH)**

**EP 0 036 835 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

Verfahren zur Herstellung von Metallkomplexen von Isoindolinazinen

In der GB-PS 1 467 595 ist die Herstellung von 1 : 1 Metallkomplexen von Azinen der Formel

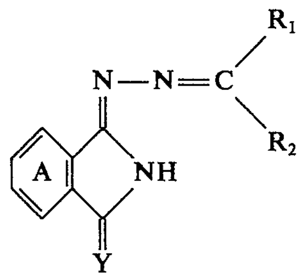
(1')

beschrieben, worin $R_1$ ein H-Atom, eine Alkyl- oder Arylgruppe, $R_2$ einen isocyclischen oder heterocyclischen Rest mit einer zur Azomethingruppe benachbarten Hydroxygruppe, Y den Rest einer methylenaktiven Verbindung, eines Aryl- oder Heteroarylamins bedeuten, und der Ring A nicht wasserlöslichmachende Substituenten aufweisen kann, durch Kondensation eines Hydrazons der Formel

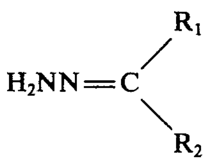
(2)

mit einem Isoindolin der Formel

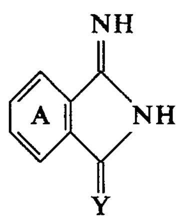
(3')

und Behandeln der Verbindungen der Formel 1') mit metallabgebenden Mitteln. Dabei erhält man die Verbindung der Formel 1') zwar in guter Ausbeute; die daraus erhaltenen 1 : 1-Nickelkomplexe ergeben jedoch in Kunststoffen und Lacken Färbungen von unbefriedigender Reinheit und ungenügenden Echtheiten. Die Kondensation des Isoindolins der Formel 3') mit dem Hydrazon der Formel 2) in Gegenwart von Metallsalzen führt ebenfalls zu keinen befriedigenden Ergebnissen.

Es wurde nun gefunden, daß man die 1 : 1-Metallkomplexe von Azinen der Formel

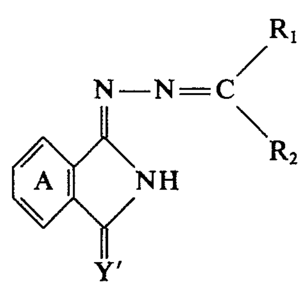
(1)

worin $R_1$ ein H-Atom, eine Alkyl- oder Arylgruppe, $R_2$ einen isocyclischen oder heterocyclischen Rest mit einer zur Azomethingruppe benachbarten Hydroxy- oder Mercaptogruppe, Y' den Rest einer methylenaktiven Verbindung oder eines Aryl- oder Heteroarylamins bedeuten, und der Ring A) nicht wasserlöslichmachende Substituenten aufweisen kann, in besonders hoher Reinheit erhält, wenn man ein Hydrazon der Formel

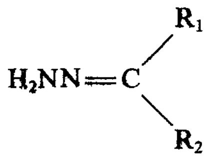

(2)

mit einem Isoindolin der Formel

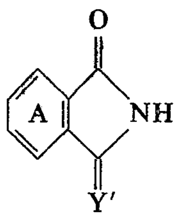

(3)

worin $R_1$, $R_2$, A und Y' die angegebene Bedeutung haben, in Gegenwart einer metallabgebenden Verbindung in einem polaren organischen Lösungsmittel erhitzt.

Die Formel 1) stellt eine der möglichen isomeren Formen dar.

Die Hydrazone der Formel 2) erhält man nach bekanntem Verfahren durch Umsetzen einer Oxoverbindung der Formel

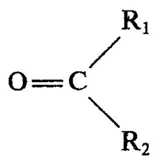

(4)

worin $R_1$ und $R_2$ die angegebene Bedeutung haben, oder deren Aldimin mit Hydrazinhydrat. Von besonderem Interesse sind Aldehyde oder Ketone der Formel

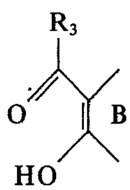

(5)

worin $R_3$ ein H-Atom oder bevorzugt eine Methylgruppe und B einen Phenyl- bzw. Naphthalinrest oder einen 5- oder 6gliedrigen Heteroring bedeuten, der in $\alpha$- oder $\gamma$-Stellung zum C-Atom, an dem die Hydroxygruppe sitzt, ein O-, S- oder insbesondere N-Atom aufweist, gegebenenfalls noch ein weiteres N-Atom im Ring und gegebenenfalls einen ankondensierten Benzolring und/oder einen weiteren Heteroring enthält.

Vorzugsweise geht man von einem Hydrazon der Formel 2) aus, worin $R_1$ ein H-Atom oder die Methylgruppe und $R_2$ einen Rest der Pyrazol-, Pyridin-, Pyrimidin-, Chinolin- oder Cumarinreihe bedeuten.

Bevorzugt sind Oxoverbindungen der Formel

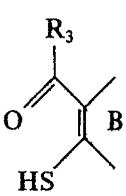

(6)

Als Beispiele von Oxoverbindungen seien die in der GB-PS 1 467 595 aufgeführten Aldehyde und Ketone genannt, ferner

1-Phenyl-3-methyl-4-formyl-5-mercapto-pyrazol,
1-(o,m- oder p-Chlorphenyl)-3-methyl-4-formyl-5-mercapto-pyrazol,
1-(o,m- oder p-Methylphenyl)-2-methyl-4-formyl-5-mercapto-pyrazol,
1-Phenyl-3-methyl-4-acetyl-5-mercapto-pyrazol,
1-(o,m- oder p-Chlorphenyl)-3-methyl-4-acetyl-5-mercapto-pyrazol,
1-(o,m- oder p-Methylphenyl)-3-methyl-4-acetyl-5-mercapto-pyrazol,

2-Formyl-5,5-dimethyl-cyclohexyl-1,3-dion, 2-Mercapto-benzaldehyd, 2-Mercapto-acetophenon, 3-Acetyl-2,4-dimercapto-chinolin, 3-Formyl-2,4-dimercapto-chinolin, 3-Formyl-2-mercapto-chinolin, 3-Formyl-2,4,6-trimercapto-pyridin, 3-Acetyl-2,4,6-trimercapto-pyrimidin, 4-Mercaptocumarin, 2-Formyl-1,3-dithiono-5,5-dimethylcyclohexan.

Besonders bevorzugt verwendet man ein Hydrazon der Formel 2), worin $R_1$ ein H-Atom oder die Methylgruppe und $R_2$ einen Rest der Formel

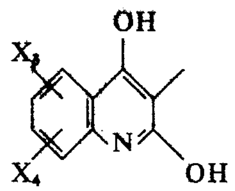

(7)

bedeuten, worin $X_3$ und $X_4$ unabhängig voneinander ein H- oder Halogenatom oder Alkyl mit 1–4 C sind.

Die Isoindolinone der Formel 3) erhält man nach bekanntem Verfahren durch Kondensation eines Isoindolinons der Formel

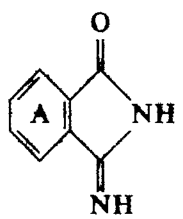

(8)

eines Phthalodinitrils oder eines o-Cyanbenzoesäureesters mit einer methylenaktiven Verbindung, einem Aryl- oder Heteroarylamin. Als Isoindolinone der Formel 3) verwendet man insbesondere solche, worin A keine weiteren Substituenten enthält und Y' einen Rest der Formel

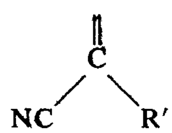

(9)

darstellt, worin R' für eine Cyangruppe, eine Alkoxycarbonyl-, Alkylcarbamoyl- oder Alkanoylgruppe mit 2–6 C, eine Benzoyl-, Carbamoyl-, oder Sulfamoylgruppe, eine Benzylcarbamoylgruppe, eine gegebenenfalls durch Halogenatome oder Alkylgruppen mit 1–4 C substituierte Phenylsulfamoyl- oder Phenylsulfonylgruppe, insbesondere aber eine Gruppe der Formeln

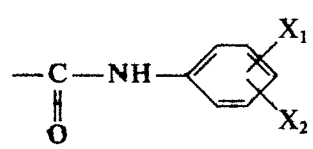

oder

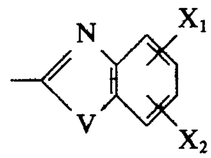

(10) (11)

steht, worin $X_1$ ein H-, Chlor- oder Bromatom, eine Nitro-, Trifluormethyl-, Carbamoyl- oder Sulfamoylgruppe, eine Alkyl-, Alkoxy- oder Alkylsulfamoylgruppe mit 1–4 C, eine Alkanoylamino-, Alkylcarbamoyl- oder Alkoxycarbonylgruppe mit 2–6 C, eine gegebenenfalls durch Chlor- oder Bromatome oder Methylgruppen substituierte Phenoxy-, Benzoylamino-, Phenylcarbamoyl-, Phenylsulfamoyl- oder Phenylazogruppe, $X_2$ ein H-, Chlor- oder Bromatom, eine Alkyl- oder Alkoxygruppe mit 1–4 C und V O, S oder NH bedeuten.

Besonders bevorzugt geht man von einem Isoindolinon der Formel 3) aus, worin Y' einen Rest der Formel

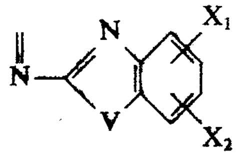

(12)

bedeutet, worin $X_1$, $X_2$ und V die oben angegebene Bedeutung haben.

Als Beispiele von methylenaktiven Verbindungen zur Herstellung der Isoindolinone der Formel 3) seien die in der GB-PS 1 467 595, Seite 7 aufgeführten Acetonitrile, ferner

Cyanessigsäure-o-chlorphenyl-, -p-chlorphenyl-, -m-chlorphenyl-, -m-methylphenyl-, -p-methylphenyl-, -3,4-dichlorphenyl-, -3,5-dimethylphenyl-, -3,4-dimethylphenyl-, -3-chlor-4-methylphenyl-, -o-methoxyphenyl-, -2,4-dimethoxyphenyl-, -2,5-dimethoxyphenyl-, -p-acetylamino-phenyl-, -p-benzoylaminophenyl-, -3-chlor-4-p-chlorbenzoylaminophenyl-, -4-carbamoylphenyl-, -4-sulfamoylphenyl-, -4-phenylazophenyl-, -4-phenoxyphenyl-, -p-nitrophenyl-, -3-trifluormethylphenyl- oder -2-chlor-5-trifluormethylphenylamid, 2-Cyanmethyl-4-phenyl-, -4-p-nitrophenyl-, -4-fluorphenyl- oder -4-methylphenylthiazol

erwähnt.

Als methylenaktive Verbindungen kommen auch Heterocyclen, die im Heteroring eine aktive Methylengruppe enthalten, in Betracht, wie sie beispielsweise auf S. 7 und 8 der GB-PS 1 467 595 erwähnt sind, beispielsweise

2,4-Dihydroxychinolin, 1-p-Chlorphenyl-3-methyl-5-pyrazolon,
1-p-Methylphenyl-3-methyl-5-pyrazolon, 1-Phenyl-3-dichlorvinyl-5-pyrazolon,
1-p-Methylphenyl-3-dichlorvinyl-5-pyrazolon.

Als Rest eines Heteroarylamins stellt Y' vorzugsweise den Rest eines derartigen Amins dar, in dem sich die Aminogruppe direkt an einem 5–6gliedrigen Heteroring befindet, der 1–3 N-Atome und außerdem noch O- und S-Atome enthalten kann. An den heterocyclischen Stammkern kann ein gegebenenfalls substituierter Benzolkern ankondensiert sein. Als Beispiele seien die in der GB-PS 1 467 595, S. 6–7 aufgeführten Amine genannt, außerdem

2-Aminopyridin, 2-Amino-5-chlorpyridin, Diaminophthalazin, 2-Amino-4-hydroxychinolin, 2,6-Diaminopyridin, 2-Amino-4,5-dimethylthiazol.

Die Isoindolinone der Formel 8) sind bekannt. Sie sind leichter zugänglich als entsprechende Diiminoisoindoline, was einen weiteren Vorzug des neuen Herstellungsverfahrens darstellt.

Als metallabgebende Mittel verwendet man vorzugsweise Salze des Zinks, Cadmiums, Mangans, Kobalts, Eisens, insbesondere aber des Kupfers und des Nickels bzw. Mischungen solcher Metalle. Besonders bevorzugt verwendet man als metallabgebendes Mittel ein Salz des Nickels. Man verwendet zweckmäßig die Formiate, Acetate oder Stearate dieser Metalle.

Die Umsetzungen erfolgen in einem polaren Lösungsmittel, insbesondere einem solchen hydrophiler Natur, beispielsweise einem Amid, wie Dimethylformamid, Formamid, Dimethylacetamid oder N-Methylpyrrolidon, ferner Dimethylsulfoxid, Acetonitril oder einem Alkohol, wie beispielsweise Äthylcellosolve. Es kann auch ein Gemisch polarer Lösungsmittel verwendet werden. Besonders bevorzugt verwendet man als polares Lösungsmittel Dimethylformamid oder N-Methylpyrrolidon. Die Umsetzungstemperatur liegt zweckmäßig zwischen 100–200° C.

Die Isolierung der erhaltenen Metall-Komplexe erfolgt wie üblich durch Filtration. Das Nutschgut wird mit Lösungsmittel gut gewaschen. Das Pigment wird in ausgezeichneter Ausbeute und Reinheit erhalten und kann ohne weitere Reinigung in feinverteilter Form zum Färben von hochmolekularem organischem Material verwendet werden, wie z. B. Celluloseäthern und -estern, wie Äthylcellulose, Acetylcellulose, Nitrocellulose, Polyamiden bzw. Polyurethanen oder Polyestern, natürlichen Harzen oder Kunstharzen, z. B. Aminoplasten, insbesondere Harnstoff- und Melamin-Formaldehydharzen, Alkydharzen, Phenoplasten, Polycarbonaten, Polyolefinen, wie Polystyrol, Polyvinylchlorid, Polyäthylen, Polypropylen, Polyacrylnitril, Polyacrylsäureestern, thermoplastischen oder härtbaren Acrylharzen, Gummi, Casein, Silikon und Silikonharzen, einzeln oder in Mischungen. Die erwähnten hochmolekularen Verbindungen können als plastische Massen, Schmelzen oder in Form von Spinnlösungen, Lacken oder Druckfarben vorliegen. Je nach Verwendungszweck erweist es sich als vorteilhaft, die Pigmente als Toner oder in Form von Präparaten zu verwenden.

Das Pigment kann in der Form, wie es in der Synthese anfällt, oder in leicht gemahlener Form eingesetzt werden und liefert dann opake Ausfärbungen. Man kann es aber auch einer intensiveren Mahlung unterziehen und erhält dann transparente Ausfärbungen, wie beispielsweise farbstarke Metalleffektlackierungen. Anreibungen der Pigmente in Lacken zeichnen sich durch ein günstiges Fließverhalten aus. Die erhaltenen Färbungen, beispielsweise in Kunststoffen, Fasern und Lacken, zeichnen sich durch große Farbstärke, hohe Farbtonreinheit, gute Dispergierbarkeit, gute Überlackier-, Migrations-, Hitze-, Licht- und Wetterechtheit sowie durch einen guten Glanz aus.

Neben der Reinheit des erhaltenen Endproduktes bestehen weitere Vorteile des erfindungsgemäßen Verfahrens darin, daß auch im Benzolring A substituierte Produkte leicht herstellbar sind und auch solche, in denen Y' den Rest einer besonders aktiven Methylengruppe bedeutet. Der Erfolg des erfindungsgemäßen Verfahrens ist überraschend, da bisher nur Umsetzungen der Carbonylfunktion

von Isoindolinen bekannt waren, bei welchen die Ketogruppe vorgängig in ein reaktionsfähiges Derivat, wie beispielsweise ein Imidchlorid oder einen Iminoäther, übergeführt wird. Es konnte daher nicht erwartet werden, daß Isoindolinone der Formel 3) direkt in einer Templatereaktion mit Hydrazonen umgesetzt werden können.

In den nachfolgenden Beispielen bedeuten die Prozente Gewichtsprozente.

Beispiel 1

6,5 g (0,03 Mol) 2,4-Dihydroxychinolin-3-acetylhydrazon und 7,8 g Nickelacetat · 4 $H_2O$ (0,315 Mol) werden in 150 ml N-Methylpyrrolidon gelöst und auf 60°C erwärmt. Anschließend gibt man 9,7 g 1-(Cyano-N-p-chlorphenylcarbamoylmethylen)-isoindolin-3-on hinzu. Die Suspension wird auf 150°C erwärmt und während 1 Stunde bei 150–155°C gerührt. Man filtriert heiß (80°C) und wäscht den Rückstand mit N-Methylpyrrolidon und Äthanol nach. Nach Trocknung bei 80°C unter Vakuum erhält man 11,34 g (65,3% der Theorie) eines roten Metallkomplexes der Zusammensetzung $C_{28}H_{17}ClN_6O_3Ni$ und der Formel

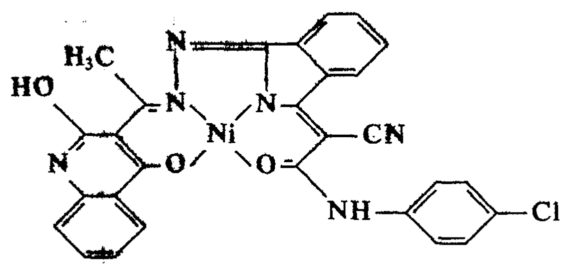

Mikroanalyse: $C_{28}H_{17}ClN_6O_3Ni$ (MG 579,65):

| | | | | | |
|---|---|---|---|---|---|
| Ber.: | C 58,02%, | H 2,96%, | N 14,50%, | Cl 6,12%, | Ni 10,13%, |
| Gef.: | C 58,1%, | H 3,2%, | N 14,7%, | Cl 5,7%, | Ni 9,95%. |

Beispiel 2

1,32 g (0,007 Mol) Dichlorsalicylaldehyd-hydrazon und 1,83 g (0,00735 Mol) Nickelacetat · 4 $H_2O$ werden in 60 ml N-Methylpyrrolidon angeschlämmt und auf 60°C erwärmt. Man gibt 2,00 g (0,007 Mol) 1-(Cyan-2-benzimidazolyl-methyl)-3-isoindolinon hinzu und erhitzt auf 150°C. Nach 2stündigem Rühren bei 150–155°C wird das Reaktionsgemisch auf 35°C abgekühlt und filtriert. Der Rückstand wird hintereinander mit N-Methylpyrrolidon und Äthanol gewaschen und über Nacht unter Vakuum bei 80°C getrocknet. Man erhält 1,4 g (36,5% der Theorie) des Nickelkomplexes der Formel

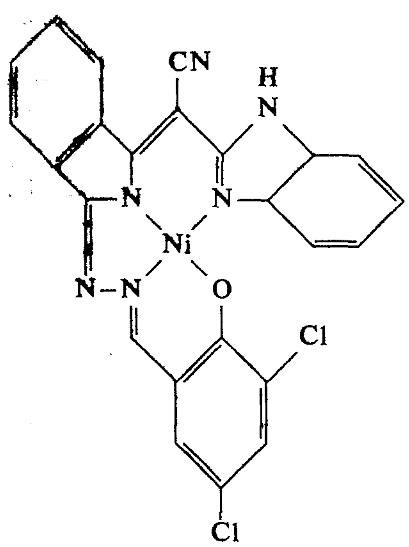

Mikroanalyse: $C_{24}H_{11}N_6OCl_2Ni$:

| | | | | | |
|---|---|---|---|---|---|
| Ber.: | C 54,49%, | H 2,10%, | N 15,89%, | Cl 13,4%, | Ni 11,10%, |
| Gef.: | C 54,6%, | H 2,3%, | N 16,1%, | Cl 13,6%, | Ni 11,4%. |

Der obige Metallkomplex färbt Kunststoffe und Lacke in roten, deckenden Tönen von ausgezeichneten Echtheiten.

Beispiel 3

1,3 g (0,006 Mol) 2,4-Dihydroxy-3-acetylchinolin-hydrazon und 1,57 g (0,0063 Mol) Nickelacetat · 4 $H_2O$ werden unter Rühren in 50 ml N-Methylpyrrolidon auf 60° C erwärmt. Hierauf gibt man 1,92 g (0,006 Mol) 1-(5'-Acetylamino-2'-benzoxazolyl-imino)-3-isoindolinon, hergestellt aus 1-Imino-3-oxo-isoindolin und 5-Acetylamino-2-aminobenzoxazol, hinzu und erwärmt das Gemisch auf 140° C. Nachdem man das Reaktionsgemisch 2,5 Stunden bei 140 – 145° C hat ausreagieren lassen, kühlt man auf 80° C ab, filtriert und wäscht das Reaktionsprodukt mit N-Methylpyrrolidon und Sprit nach. Nach Trocknung bei 80° C unter Vakuum erhält man 3,06 g (88,5% der Theorie) eines orangen Metallkomplexes der Zusammensetzung $C_{28}H_{19}N_7O_4Ni$ und der Formel

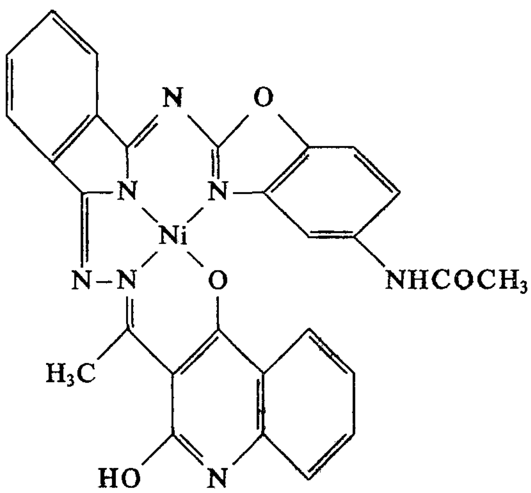

Mikroanalyse: $C_{28}H_{19}N_7O_4Ni$ MG 576:

| | | | | |
|---|---|---|---|---|
| Ber.: | C 58,36%, | H 3,32%, | N 17,02%, | Ni 10,19%, |
| Gef.: | C 58,2%, | H 3,5%, | N 17,2%, | Ni 10,0%. |

Das obige Pigment färbt Kunststoffe und Lacke in gelb-orangen Tönen von ausgezeichneten Echtheiten.

Beispiel 4

1,75 g (0,008 Mol) 4-Hydroxy-3-acetyl-cumarin-hydrazon und 2,1 g (0,0084 Mol) Nickelacetat werden in 60 ml N-Methylpyrrolidon angeschlämmt und anschließend auf 60° C erwärmt. Die resultierende Suspension wird mit 2,32 g (0,008 Mol) 1-(2',4'-Dioxo-chinolin-3'-yliden)-3-isoindolinon, hergestellt aus 1-Imino-3-oxoisoindolin und 2,4-Dihydroxychinolin, versetzt und auf 140° C erhitzt. Man läßt das Gemisch bei 140 – 145° C 2 h ausreagieren, filtriert anschließend den ausgefallenen Metallkomplex von der Mutterlauge ab. Nach Waschen mit N-Methylpyrrolidon und Sprit und Trocknung unter Vakuum bei 80° C erhält man 3,0 g (68,5% der Theorie) eines roten Metallkomplex-Pigmentes der Formel

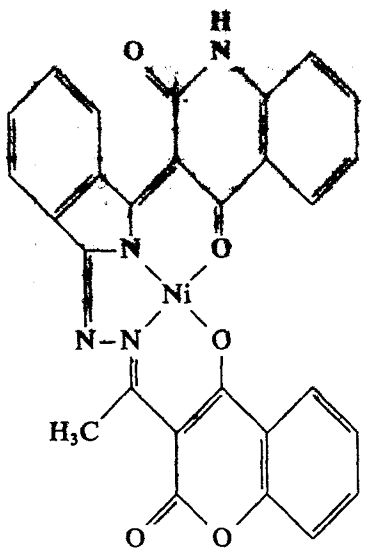

Der obige Metallkomplex färbt Kunststoffe und Lacke in reinen roten Tönen von ausgezeichneten Echtheiten.

Mikroanalyse: $C_{27}H_{14}N_4O_5Ni$, MG 533:

| | | | | |
|---|---|---|---|---|
| Ber.: | C 60,83%, | H 2,65%, | N 10,51%, | Ni 11,01%, |
| Gef.: | C 60,9%, | H 2,4%, | N 10,8%, | Ni 10,9%. |

Beispiel 5

1,68 g (0,006 Mol) 2,4-Dihydroxy-3-benzoylchinolinhydrazon und 1,57 g (0,0063 Mol) Nickelacetat · 4 $H_2O$ werden in 50 ml N-Methylpyrrolidon angeschlämmt und auf 60° C erwärmt. Anschließend gibt man 1,95 g (0,006 Mol) 1-(Cyano-N-p-chlorphenylcarbamoylmethylen)-isoindolin-3-on hinzu. Die Suspension wird auf 145° C erwärmt und während 1½ h bei 145–150° C gerührt. Man filtriert heiß (80° C) und wäscht den Rückstand mit N-Methylpyrrolidon und Äthanol nach. Nach Trocknung bei 80° C unter Vakuum erhält man 2,34 g (61% der Theorie) eines roten Metallkomplexes der Formel

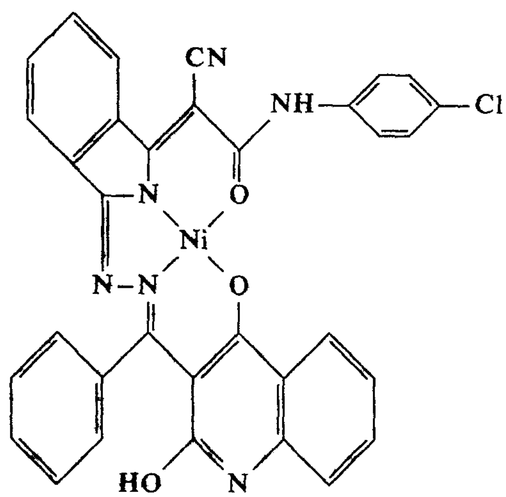

Mikroanalyse: $C_{33}H_{19}ClN_6O_3Ni$, MG 642:

| | | | | | |
|---|---|---|---|---|---|
| Ber.: | C 61,77%, | H 2,98%, | N 13,1%, | Cl 5,53%, | Ni 9,15%, |
| Gef.: | C 61,2%, | H 3,00%, | N 13,1%, | Cl 5,4%, | Ni 9,42%. |

Das obige Metallkomplex-Pigment färbt Kunststoffe und Lacke in reinen roten Tönen von ausgezeichneter Echtheit.

Beispiel 6

1,95 g (0,008 Mol) 2-Anilinomethylen-1,3-dioxo-5,5-dimethyl-cyclohexan, hergestellt aus Anilin, Orthoameisensäureester und 1,3-Dioxo-5,5-dimethyl-cyclohexan, werden in 50 ml Dimethylformamid gelöst. Nach Zugabe von 0,4 ml (0,008 Mol) Hydrazinhydrat wird das Gemisch bei Raumtemperatur 1 h gerührt. Anschließend gibt man 2,1 g (0,0084 Mol) Nickelacetat · 4 $H_2O$ hinzu und erwärmt auf 60°C. Nun wird das Gemisch mit 2,29 g (0,008 Mol) 1-(Cyan-benzimidazolyl-methylen)-3-isoindolinon versetzt und auf 135°C erhitzt. Nach 2½ h wird das Reaktionsgemisch auf 80°C abgekühlt und der ausgefallene Metallkomplex abfiltriert. Nach Waschen mit Dimethylformamid und Sprit, sowie Trocknung bei 80°C unter Vakuum erhält man 3,5 g (86,3% der Theorie) einer orangen Verbindung der Formel

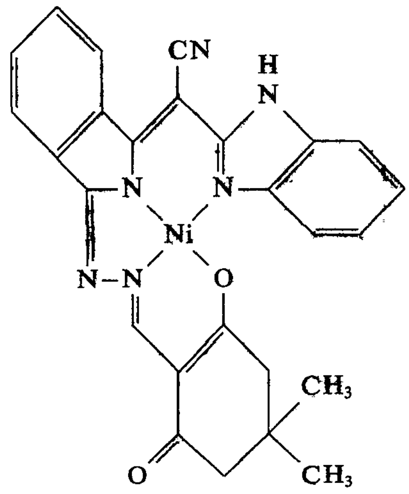

Mikroanalyse: $C_{26}H_{20}N_6O_2Ni$, MG 507,2:

| | | | | |
|---|---|---|---|---|
| Ber.: | C 61,57%, | H 3,97%, | N 16,57%, | Ni 11,58%, |
| Gef.: | C 61,4%, | H 4,1%, | N 16,8%, | Ni 11,6%. |

Der obige Metallkomplex färbt Kunststoffe und Lacke in reinen gelborangen Tönen von ausgezeichneten Echtheiten.

Beispiel 7

1,95 g (0,008 Mol) 2-Anilinomethylen-1,3-dioxo-5,5-dimethyl-cyclohexan werden in 50 ml N-Methylpyrrolidon gelöst, mit 0,4 ml (0,008 Mol) Hydrazinhydrat versetzt und bei Raumtemperatur 1 h gerührt. Man gibt 2,1 g (0,0084 Mol) Nickelacetat · 4 $H_2O$ hinzu und erwärmt auf 60°C. Anschließend trägt man 3,2 g (0,008 Mol) 1-Benzimidazolylimino-4,5,6,7-tetrachlor-3-oxo-isoindolin ein und erwärmt auf 145°C. Das Gemisch wird 1 h bei derselben Temperatur gerührt und anschließend abgekühlt (auf 80°C). Der ausgefallene Metallkomplex wird abfiltriert, mit N-Methylpyrrolidon und Äthanol gewaschen und über Nacht bei 80°C unter Vakuum getrocknet. Man erhält 3,9 g (78,5% der Theorie) eines roten Pigments der Formel

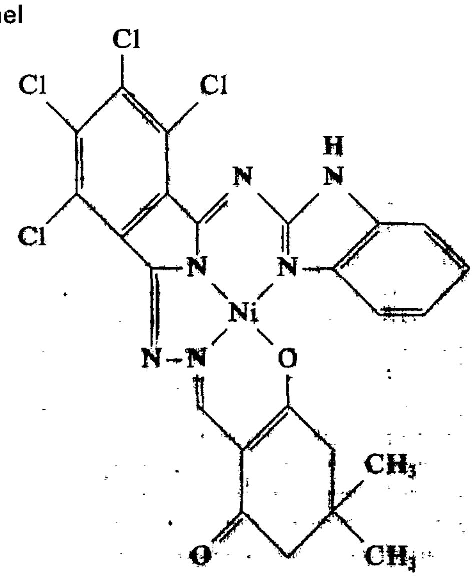

das Kunststoffe und Lacke in roten Tönen von ausgezeichneten Echtheiten färbt.

Mikroanalyse: $C_{24}H_{16}Cl_4N_6O_2Ni$, MG 621:

| | | | | | |
|---|---|---|---|---|---|
| Ber.: | C 46,42%, | H 2,60%, | N 13,53%, | Cl 22,84%, | Ni 9,45%, |
| Gef.: | C 46,7%, | H 2,7%, | N 13,8%, | Cl 22,3%, | Ni 9,36%. |

Beispiel 8

2,35 g (0,008 Mol) 1-Phenyl-3-methyl-4-anilinomethylen-5-mercaptopyrazol werden in 50 ml Dimethylformamid gelöst. Nach Zugabe von 0,4 ml (0,008 Mol) Hydrazinhydrat wird das Gemisch bei Raumtemperatur 1 h gerührt, anschließend mit 2,1 g (0,0084 Mol) Nickelacetat · 4 $H_2O$ versetzt und auf 60°C erwärmt. Nun trägt man 2,22 g (0,008 Mol) 1-(Cyan-benzimidazolylmethylen)-3-oxo-isoindolin ein und erhitzt auf 130°C. Nach einer Reaktionszeit von 2 h bei 130–135°C wird das Gemisch auf 80°C abgekühlt und filtriert. Der Filterrückstand wird mit Dimethylformamid und Äthanol gewaschen und über Nacht bei 80°C unter Vakuum getrocknet. Man erhält 2,8 g (63% der Theorie) eines roten Metallkomplexes der Formel

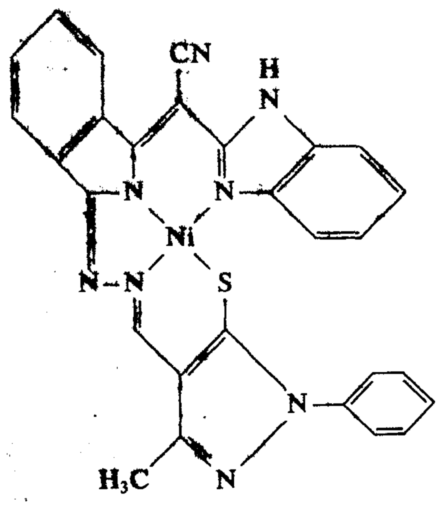

Mikroanalyse: $C_{28}H_{18}N_8SNi$, MG 557:

| | | | | | |
|---|---|---|---|---|---|
| Ber.: | C 60,35%, | H 3,26%, | N 20,11%, | S 5,75%, | Ni 10,54%, |
| Gef.: | C 60,3%, | H 3,4%, | N 20,0%, | S 5,2%, | Ni 10,3%. |

Beispiele 9–76

Analog den Beispielen 1–8 erhält man weitere 1 : 1-Nickelkomplexe, wenn man das Hydrazon der in Kolonne 2 der Tabelle 1 angegebenen Oxoverbindungen mit dem Isoindolinon der Formel

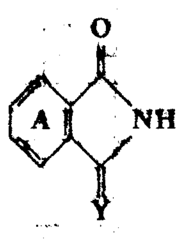

kondensiert. Das letztere wird durch Kondensation des in Kolonne 3 erwähnten 3-Imino-isoindolinons mit der in Kolonne 4 aufgeführten Verbindung $YH_2$ erhalten. Kolonne 5 gibt die Nuance in PVC an.

Tabelle 1

| Beispiel Nr. | Oxoverbindung | Isoindolinon | $YH_2$ | Nuance in PVC |
|---|---|---|---|---|
| 9 | 1-p-Tolyl-3-methyl-4-acetylpyrazolon-5 | 3-Imino-isoindolinon | Cyanacetanilid | rot |
| 10 | 1-p-Chlorphenyl-3-methyl-4-formyl-pyrazolon-5 | desgl. | Cyanessigsäure-p-chlor-anilid | scharlach |
| 11 | 3-Acetyl-2,4-di-hydroxychinolin | desgl. | Cyanessigsäureäthylester | rot |
| 12 | desgl. | desgl. | Cyanessigsäureäthylamid | rot |
| 13 | desgl. | desgl. | Acetessigsäurenitril | rot |
| 14 | desgl. | desgl. | Cyanessigsäure-p-methoxyanilid | rot |
| 15 | desgl. | desgl. | Cyanessigsäurebenzyl-amid | rot |
| 16 | desgl. | desgl. | Benzoylacetonitril | rot |
| 17 | desgl. | desgl. | Cyanmethan-p-tolyl-sulfonamid | rot |
| 18 | desgl. | desgl. | Cyanmethan-p-chlor-phenylsulfonamid | rot |
| 19 | desgl. | desgl. | Cyanessigsäure-p-sulf-amoylanilid | rot |
| 20 | desgl. | desgl. | Cyanessigsäure-p-carb-amoylanilid | rot |
| 21 | desgl. | desgl. | Phenylsulfonylacetonitril | rot |
| 22 | desgl. | desgl. | Cyanessigsäure-p-benzoylaminoanilid | rot |
| 23 | desgl. | desgl. | Cyanessigsäure-p-acetyl-aminoanilid | rot |
| 24 | desgl. | 3-Imino-4,5,6,7-tetra-chlor-isoindolinon-1 | Cyanacet-p-chlor-anilid | orange |
| 25 | desgl. | 3-Imino-5,7-di-methoxy-4,6-dichlor-isoindolinon | Cyanessigsäure-p-chlor-anilid | orange |
| 26 | desgl. | 3-Imino-4,5,6,7-tetra-chlor-isoindolinon | 2-Cyanmethyl-benzimid-azol | violett |
| 27 | desgl. | 3-Imino-isoindolinon | Cyanessigsäure-m-chlor-anilid | rot |
| 28 | desgl. | desgl. | Cyanessigsäure-3',4'-di-chloranilid | scharlach |

Fortsetzung

| Beispiel Nr. | Oxoverbindung | Isoindolinon | $YH_2$ | Nuance in PVC |
|---|---|---|---|---|
| 29 | 3-Acetyl-2,4-dihydroxychinolin | 3-Imino-isoindolinon | 2-Cyanmethylbenzimidazol | rot |
| 30 | desgl. | desgl. | 2-Cyanmethylbenzthiazol | rot |
| 31 | desgl. | desgl. | 2-Cyanmethylbenzoxazol | scharlach |
| 32 | desgl. | desgl. | Cyanacetanilid | rot |
| 33 | 1-p-Chlorphenyl-3-methyl-4-acetyl-pyrazolon-5 | desgl. | Cyanessigsäure-p-acetyl-amino-anilid | rot |
| 34 | 3-Acetyl-2,4-dihydroxychinolin | desgl. | Cyanessigsäure-p-nitro-anilid | rot |
| 35 | desgl. | desgl. | Cyanessigsäure-p-phenylazoanilid | rot |
| 36 | desgl. | desgl. | Cyanessigsäure-m-trifluormethylanilid | scharlach |
| 37 | desgl. | desgl. | Cyanessigsäure-p-phenoxyanilid | rot |
| 38 | desgl. | desgl. | Cyanessigsäure-3'-chlor-4'-methylanilid | rot |
| 39 | desgl. | desgl. | Cyanessigsäureacetyl-aminoanilid | rot |
| 40 | desgl. | desgl. | Cyanessigsäure-p-phenyl-sulfamoyl-anilid | rot |
| 41 | desgl. | desgl. | Cyanessigsäure-p-phenyl-carbamoyl-anilid | rot |
| 42 | desgl. | desgl. | Cyanessigsäure-o-äthoxy-carbonylanilid | rot |
| 43 | desgl. | desgl. | 2-Cyanmethyl-4-p-fluorphenylthiazol | rot |
| 44 | desgl. | 3-Imino-5,6-dichlor-isoindolinon | Barbitursäure | orange |
| 45 | desgl. | 3-Imino-isoindolinon | 1-p-Tolyl-3-$\beta,\beta$-dichlor-vinylpyrazolon-5 | bordeaux |
| 46 | 3-Acetyl-2,4-dihydroxychinolin | desgl. | Barbitursäure | rot |
| 47 | desgl. | desgl. | 1-Phenyl-3-methyl-pyrazolon-5 | rot |
| 48 | desgl. | 3-Imino-4,5,6,7-tetra-chlor-isoindolinon | Barbitursäure | orange |

Fortsetzung

| Beispiel Nr. | Oxoverbindung | Isoindolinon | $YH_2$ | Nuance in PVC |
|---|---|---|---|---|
| 49 | 3-Acetyl-2,4-dihydroxychinolin | 3-Imino-4,5,6,7-tetrachlor-isoindolinon | 2-Cyanmethylbenzimidazol | violett |
| 50 | desgl. | desgl. | Cyanessigsäure-2',5'-dimethoxyanilid | rot |
| 51 | desgl. | desgl. | Cyanessigsäure-3',4'-dimethylanilid | rot |
| 52 | 2,4,6-Trihydroxy-5-acetylpyrimidin | 3-Imino-4,5,6,7-tetrachlor-isoindolinon | 2-Aminoimidazol | orange |
| 53 | 3-Acetyl-2,4-dihydroxychinolin | desgl. | 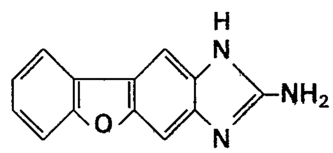 | orange |
| 54 | desgl. | desgl. | 2-Amino-5-methyl-benzoylamino-benzimidazol | orange |
| 55 | desgl. | desgl. | 2-Amino-5-p-chlor-benzoylamino-benzimidazol | orange |
| 56 | desgl. | desgl. | 2-Amino-5-benzoyl-amino-benzimidazol | scharlach |
| 57 | desgl. | 3-Imino-5,6-dichlor-isoindolinon | 2-Amino-benzthiazol | orange |
| 58 | desgl. | 3-Imino-isoindolinon | 2-Aminothiazol | orange |
| 59 | desgl. | 3-Imino-5,6-dichlor-isoindolinon | 2-Amino-5-äthoxy-benzimidazolon | rot |
| 60 | desgl. | 3-Imino-4,5,6,7-tetrachlor-isoindolinon | 2-Amino-benzthiazol | rot |
| 61 | desgl. | desgl. | 2-Amino-benzimidazol | rot |
| 62 | 3-Acetyl-4-hydroxy-cumarin | desgl. | desgl. | orange |
| 63 | 3-Acetyl-2,4-dihydroxychinolin | 3-Imino-isoindolinon | desgl. | orange |
| 64 | desgl. | desgl. | desgl. | orange |
| 65 | desgl. | desgl. | 1,4-Diaminophthalazin | scharlach |
| 66 | 1-Phenyl-3-methyl-4-formyl-5-mercapto-pyrazol | desgl. | Cyanacet-anilid | rot |
| 67 | desgl. | desgl. | Cyanessigsäure-4'-phenoxyanilid | rot |

Fortsetzung

| Beispiel Nr. | Oxoverbindung | Isoindolinon | $YH_2$ | Nuance in PVC |
|---|---|---|---|---|
| 68 | 1-Phenyl-3-methyl-4-formyl-5-mercapto-pyrazol | 3-Imino-isoindolinon | Cyanessigsäure-4'-acetylaminoanilid | rot |
| 69 | desgl. | desgl. | Cyanessigsäure-4'-methylanilid | rot |
| 70 | desgl. | desgl. | Cyanessigsäure-4'-methoxyanilid | rot |
| 71 | desgl. | desgl. | Cyanessigsäure-4'-chloranilid | rot |
| 72 | 1-p-Chlorphenyl-3-methyl-4-acetyl-pyrazolon-5 | 3-Imino-4,6-dimethoxy-5,7-dichlor-isoindolinon | Cyanacetamid | bordeaux |
| 73 | desgl. | 3-Imino-5,6-dichlor-iso-indolinon | desgl. | rot |
| 74 | desgl. | 3-Imino-4,6-dimethoxy-5,7-dichlor-isoindolinon | desgl. | bordeaux |
| 75 | 3-Acetyl-4-hydroxy-cumarin | desgl. | desgl. | rot |
| 76 | desgl. | 3-Imino-5,6-dichlor-iso-indolinon | desgl. | rot |
| 77 | 1-Phenyl-3-alkoxy-carbonyl-4-formyl-5-mercaptopyrazol | 3-Imino-isoindolinon | Cyanmethyl-benzimidazol | rot |
| 78 | 1-Phenyl-3-thio-carbamoyl-4-formyl-5-mercaptopyrazol | desgl. | 2-Amino-benzimidazol | orange |
| 79 | 7-Chloro-4-methoxy-3-formyl-2-mercapto-chinolin | desgl. | Cyanacet-p-chloranilid | rot |

Beispiele 80–82

Durch Umsetzung analog den Beispielen 1–8 des Isoindolinon-Derivats der Formel

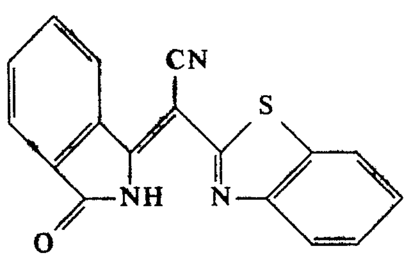

mit dem Hydrazon der Formel

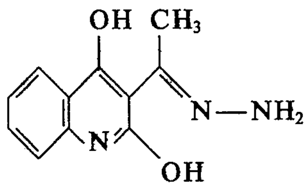

in Gegenwart des Acetates des in Kolonne 2 der Tabelle 2 aufgeführten Metalles, erhält man weitere Metallkomplexe der Formel

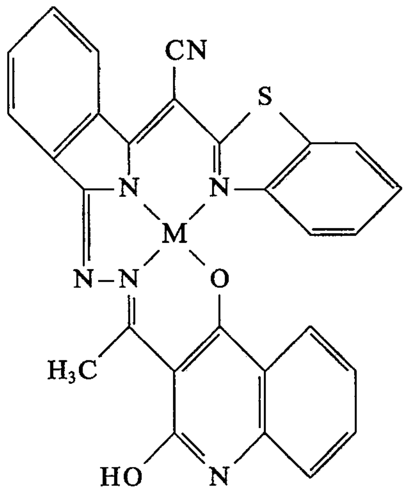

(der Einfachheit halber wird nur eine der möglichen isomeren oder tautomeren Formen berücksichtigt), wobei M das in Kolonne 2 angegebene Metall darstellt. Kolonne 3 gibt die Nuance der Färbung in Polyvinylchlorid wieder.

Tabelle 2

| Beispiel Nr. | M | Nuance in PVC |
|---|---|---|
| 80 | Pd | rot |
| 81 | Cu | rot |
| 82 | Zn | scharlachrot |

Beispiel 83

In einem Laboratoriums-Kneter von 250 Vol.-Teilen Inhalt legt man 25 Teile des gemäß Beispiel 1 hergestellten Pigmentes, 100 Teile feingemahlenes Natriumchlorid und 30 Teile Diacetonalkohol vor. Man knetet die Mischung während 5 Stunden unter Kühlung und trägt sie dann in 4000 Vol.-Teile Wasser ein. Natriumchlorid sowie Diacetonalkohol gehen in Lösung, das Pigment fällt aus. Man filtriert die Suspension, wäscht das Nutschgut gründlich mit Wasser und trocknet es im Vakuumtrockenschrank bei 80° C.

Beispiel 84

65 Teile stabilisiertes Polyvinylchlorid, 35 Teile Dioctylphthalat und 0,2 Teile des gemäß Beispiel 76 erhaltenen Pigmentes werden miteinander verrührt und dann auf einem Zweiwalzenkalander während 7 Minuten bei 140° C hin- und hergewalzt. Man erhält eine rote gefärbte Folie von sehr guter Licht- und Migrationsechtheit.

Beispiel 85

10 g Titandioxyd und 2 g des nach Beispiel 76 hergestellten Pigments werden mit 88 g einer Mischung von 26,4 g Kokosalkydharz, 24,0 g Melamin-Formaldehydharz (50% Festkörpergehalt), 8,8 g Äthylenglykolmonomethyläther und 28,8 g Xylol während 48 Stunden in einer Kugelmühle vermahlen.

Wird dieser Lack auf eine Aluminiumfolie gespritzt, 30 Minuten bei Raumtemperatur vorgetrocknet und dann während 30 Minuten bei 120°C eingebrannt, dann erhält man eine Rotlackierung, die sich bei guter Farbstärke durch eine sehr gute Überlackier-, Licht- und Wetterechtheit auszeichnet.

Beispiel 86

4 Teile des fein verteilten Pigments gemäß Beispiel 76 werden in 20 Teilen Lösungsmittel der folgenden Zusammensetzung eingerührt: 50 Teile Solvesso 150® (Gemisch aromatischer Kohlenstoffe), 15 Teile Butylacetat, 5 Teile Exkin II® (Verlaufmittel auf Ketoximbasis), 25 Teile Methyl-Isobutylketon, 5 Teile Silikonöl (1% in Solvesso 150).

Nachdem die vollständige Feinverteilung erreicht ist (je nach Art des Rührers in ca. 15 – 60 Minuten), werden die Bindemittel zugesetzt, nämlich 48,3 Teile Baycryl L 530® (Acrylharz) (51% in Xylol/Butanol 3 : 1) und 23,7 Teile Maprenal TTX® (Melaminharz) (55% in Butanol).

Nach kurzem Homogenisieren wird der Lack nach üblichen Methoden, wie Spritzen und Tauchen oder speziell zur kontinuierlichen Beschichtung von Metallblechen im »Coil-Coating«-Verfahren, appliziert und eingebrannt (Einbrennen 30 Minuten, 130°C). Die erhaltenen roten Lackierungen zeichnen sich aus durch sehr guten Verlauf, hohen Glanz und ausgezeichnete Feinverteilung des Pigmentes, sowie durch ausgezeichnete Wetterechtheiten.

Beispiel 87

Verfährt man wie in Beispiel 83 beschrieben, fügt jedoch der Knetmasse 2,78 Teile Staybelite Resin® (HERCULES) zu, so erhält man ein Pigment, enthaltend 10% Harz, das sich durch leichtere Einarbeitbarkeit und bessere Verteilbarkeit auszeichnet.

**Patentansprüche**

1. Verfahren zur Herstellung von 1 : 1-Metallkomplexen von Azinen der Formel

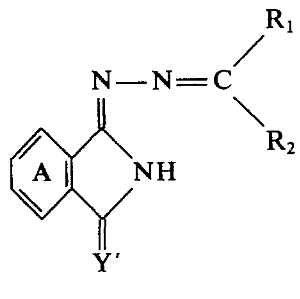

(1)

worin $R_1$ ein H-Atom, eine Alkyl- oder Arylgruppe, $R_2$ einen isocyclischen oder heterocyclischen Rest mit einer zur Azomethingruppe benachbarten Hydroxy- oder Mercaptogruppe, Y' den Rest einer methylenaktiven Verbindung, eines Aryl- oder Heteroarylamins bedeuten, und der Ring A nicht wasserlöslichmachende Substituenten aufweisen kann, dadurch gekennzeichnet, daß man ein Hydrazon der Formel

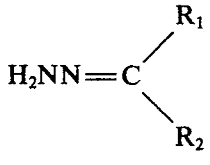

(2)

mit einem Isoindolinon der Formel

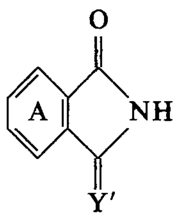

(3)

worin $R_1$, $R_2$, A und Y' die angegebene Bedeutung haben, in Gegenwart einer metallabgebenden Verbindung in einem polaren organischen Lösungsmittel erhitzt.

2. Verfahren gemäß Anspruch 1, dadurch gekennzeichnet, daß man von einem Hydrazon der Formel 2) ausgeht, worin $R_1$ ein H-Atom oder die Methylgruppe und $R_2$ einen Rest der Pyrazol-, Pyridin-, Pyrimidin-, Chinolin- oder Cumarinreihe bedeuten.

3. Verfahren gemäß Anspruch 1, dadurch gekennzeichnet, daß man von einem Hydrazon der Formel 2) ausgeht, worin $R_1$ ein H-Atom oder die Methylgruppe und $R_2$ einen Rest der Formel

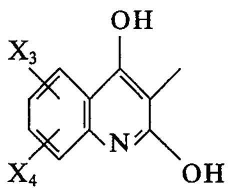

(7)

bedeuten, worin $X_3$ und $X_4$ unabhängig voneinander ein H- oder Halogenatom oder Alkyl mit 1–4 C sind.

4. Verfahren gemäß Anspruch 1, dadurch gekennzeichnet, daß man von einem Isoindolinon der Formel 3) ausgeht, worin A keine weiteren Substituenten enthält und Y' einen Rest der Formel

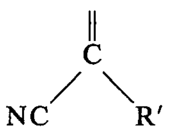

(9)

bedeutet, worin R' für eine Cyangruppe, eine Alkoxycarbonyl-, Alkylcarbamoyl- oder Alkanoylgruppe mit 2–6 C, eine Benzoyl-, Carbamoyl- oder Sulfamoylgruppe, eine Benzylcarbamoylgruppe, eine gegebenenfalls durch Halogenatome oder Alkylgruppen mit 1–4 C substituierte Phenylsulfamoyl- oder Phenylsulfonylgruppe, insbesondere aber eine Gruppe der Formel

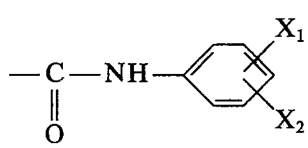

oder

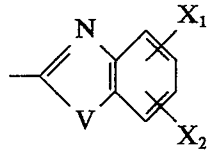

(10) (11)

steht, worin $X_1$ ein H-, Chlor- oder Bromatom, eine Nitro-, Trifluormethyl-, Carbamoyl- oder Sulfamoylgruppe, eine Alkyl-, Alkoxy- oder Alkylsulfamoylgruppe mit 1–4 C, eine Alkanoylamino-, Alkylcarbamoyl- oder Alkoxycarbonylgruppe mit 2–6 C, eine gegebenenfalls durch Chlor- oder Bromatome oder Methylgruppen substituierte Phenoxy-, Benzoylamino-, Phenylcarbamoyl-, Phenylsulfamoyl- oder Phenylazogruppe, $X_2$ ein H-, Chlor- oder Bromatom, eine Alkyl- oder Alkoxygruppe mit 1–4 C und V O, S oder NH bedeuten.

5. Verfahren gemäß Anspruch 1, dadurch gekennzeichnet, daß man von einem Isoindolinon der Formel 3) ausgeht, worin Y' den Rest der Formel

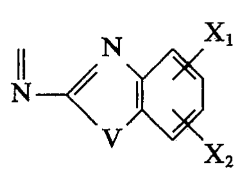

(12)

bedeutet, worin $X_1$ ein H-, Chlor- oder Bromatom, eine Nitro-, Trifluormethyl-, Carbamoyl- oder Sulfamoylgruppe, eine Alkyl-, Alkoxy- oder Alkylsulfamoylgruppe mit 1–4 C, eine Alkanoylamino-, Alkylcarbamoyl- oder Alkoxycarbonylgruppe mit 2–6 C, eine gegebenenfalls durch Chlor- oder Bromatome oder Methylgruppen substituierte Phenoxy-, Benzoylamino-, Phenylcarbamoyl-, Phenylsulfamoyl- oder Phenylazogruppe, $X_2$ ein H-, Chlor- oder Bromatom, eine Alkyl- oder Alkoxygruppe mit 1–4 C und V O, S oder NH bedeuten.

6. Verfahren gemäß Anspruch 1, dadurch gekennzeichnet, daß man als metallabgebendes Mittel ein Salz des Nickels verwendet.

7. Verfahren gemäß Anspruch 1, dadurch gekennzeichnet, daß man als polares Lösungsmittel Dimethylformamid oder N-Methylpyrrolidon verwendet.

8. Verfahren gemäß Anspruch 1, dadurch gekennzeichnet, daß man die Umsetzung bei Temperaturen zwischen 100–200° C durchführt.

## Claims

1. A process for the preparation of a 1 : 1 metal complex of an azine of the formula

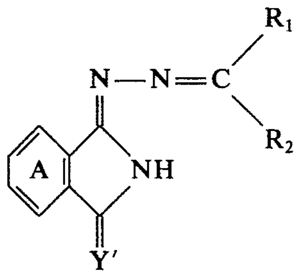

(1)

in which $R_1$ is an H atom or an alkyl or aryl group, $R_2$ is an isocyclic or heterocyclic radical with a hydroxyl or mercapto group adjacent to the azomethine group, Y′ is the radical of a methylene-active compound or of an aryl- or heteroaryl-amine and the ring A can have substituents which do not confer solubility in water, which comprises heating a hydrazone of the formula

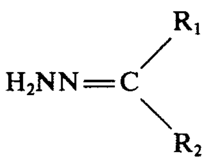

(2)

with an isoindolinone of the formula

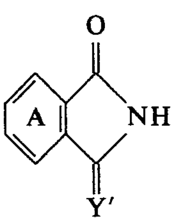

(3)

in which $R_1$, $R_2$, A and Y′ are as defined, in the presence of a metal donor, in a polar organic solvent.

2. A process according to claim 1, which comprises starting from a hydrazone of the formula 2) in which $R_1$ is an H atom or the methyl group and $R_2$ is a radical of the pyrazole, pyridine, pyrimidine, quinoline or coumarin series.

3. A process according to claim 1, which comprises starting from a hydrazone of the formula 2) in which $R_1$ is an H atom or the methyl group and $R_2$ is a radical of the formula

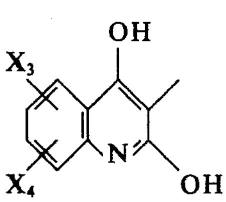

(7)

in which $X_3$ and $X_4$ independently of one another are H, Hal or alkyl having 1–4 C.

4. A process according to claim 1, which comprises starting from an isoindolinone of the formula 3) in which A does not contain any further substituents and Y' is a radical of the formula

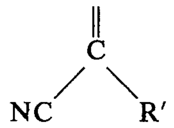

(9)

in which R' is a cyano group, an alkoxycarbonyl, alkylcarbamoyl or alkanoyl group having 2–6 C, a benzoyl, carbamoyl or sulfamoyl group, a benzylcarbamoyl group or a phenylsulfamoyl or phenylsulfonyl group which is unsubstituted or substituted by halogen atoms or alkyl groups having 1–4 C, but especially a group of the formula

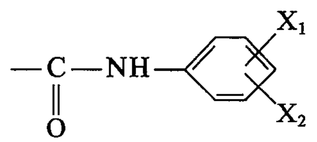

or

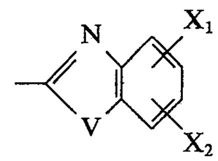

(10) (11)

in which $X_1$ is an H, chlorine or bromine atom, a nitro, trifluoromethyl, carbamoyl or sulfamoyl group, an alkyl, alkoxy or alkylsulfamoyl group having 1–4 C, an alkanoylamino, alkylcarbamoyl or alkoxycarbonyl group having 2–6 C, or a phenoxy, benzoylamino, phenylcarbamoyl, phenylsulfamoyl or phenylazo group which is unsubstituted or substituted by chlorine or bromine atoms or methyl groups, $X_2$ is an H, chlorine or bromine atom or an alkyl or alkoxy group having 1–4 C and V is O, S or NH.

5. A process according to claim 1, which comprises starting from an isoindolinone of the formula 3) in which Y' is the radical of the formula

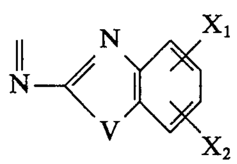

(12)

in which $X_1$ is an H, chlorine or bromine atom, a nitro, trifluoromethyl, carbamoyl or sulfamoyl group, an alkyl, alkoxy or alkylsulfamoyl group having 1–4 C, an alkanoylamino, alkylcarbamoyl or alkoxycarbonyl group having 2–6 C, or a phenoxy, benzoylamino, phenylcarbamoyl, phenylsulfamoyl or phenylazo group which is unsubstituted or substituted by chlorine or bromine atoms or methyl groups, $X_2$ is an H, chlorine or bromine atom or an alkyl or alkoxy group having 1–4 C and V is O, S or NH.

6. A process according to Claim 1, which comprises using a nickel salt as the metal donor.

7. A process according to Claim 1, which comprises using dimethylformamide or N-methylpyrrolidone as the polar solvent.

8. A process according to Claim 1, which comprises carrying out the reaction at temperatures of between 100 and 200° C.

**Revendications**

1. Procédé de préparation de complexes métalliques 1 : 1 d'azines répondant à la formule:

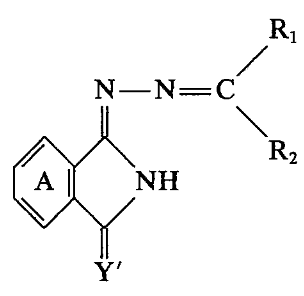

(1)

dans laquelle $R_1$ représente un atome d'hydrogène ou un radical alkyle ou aryle, $R_2$ représente un

radical isocyclique ou hétérocyclique qui porte un groupe hydroxy ou mercapto voisin du radical azométhinique, Y′ représente le radical d'un composé à méthylène actif ou le radical d'une arylamine ou d'une hétéroarylamine, et le noyau A peut porter des substituants non-hydrosolubilisants, procédé caractérisé en ce qu'on chauffe une hydrazone de formule:

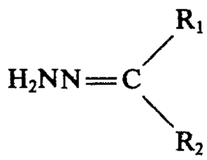

(2)

avec une iso-indolinone de formule:

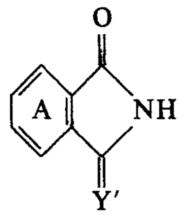

(3)

formules dans dans lesquelles $R_1$, $R_2$, A et Y′ ont les significations précédemment données, en présence d'un composé apte à céder un métal, dans un solvant organique polaire.

2. Procédé selon la revendication 1, caractérisé en ce qu'on part d'une hydrazone de formule 2), dans laquelle $R_1$ représente un atome d'hydrogène ou un radical méthyle, et $R_2$ représente un radical de la série du pyrazole, de la pyridine, de la pyrimidine, de la quinoléine ou de la coumarine.

3. Procédé selon la revendication 1, caractérisé en ce qu'on part d'une hydrazone de formule 2), dans laquelle $R_1$ représente un atome d'hydrogène ou un radical méthyle et $R_2$ représente un radical répondant à la formule:

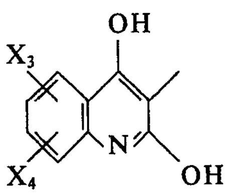

(7)

dans laquelle $X_3$ et $X_4$ représentent chacun, indépendamment l'un de l'autre, un atome d'hydrogène ou d'halogène, ou un radical alkyle contenant de 1 à 4 atomes de carbone.

4. Procédé selon la revendication 1, caractérisé en ce qu'on part d'une iso-indolinone de formule 3), dans laquelle A ne porte pas de substituant supplémentaire et Y′ représente un radical répondant à la formule:

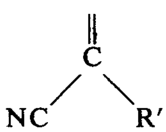

(9)

dans laquelle R′ représente un radical cyano, un radical alcoxycarbonyle, alkylcarbamoyle ou alcanoyle contenant de 2 à 6 atomes de carbone, un radical benzoyle, carbamoyle ou sulfamoyle, un radical benzylcarbamoyle, un radical phénylsulfamoyle ou phénylsulfonyle éventuellement porteur d'atomes d'halogènes ou de radicaux alkyles en $C_1-C_4$, mais plus particulièrement un radical répondant à l'une des formules:

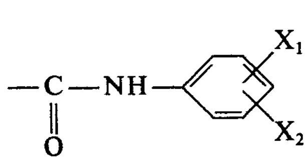

et

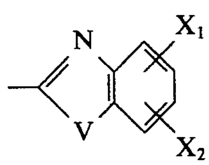

(10) (11)

dans lesquelles $X_1$ représente un atome d'hydrogène, de chlore ou de brome, un radical nitro, trifluorométhyle, carbamoyle ou sulfamoyle, un radical alkyle, alcoxy ou alkylsulfamoyle contenant de

1 à 4 atomes de carbone, un radical alcanoylamino, alkylcarbamoyle ou alcoxycarbonyle contenant de 2 à 6 atomes de carbone, ou un radical phénoxy, benzoylamino, phénylcarbamoyle, phénylsulfamoyle ou phénylazo éventuellement porteur d'atomes de chlore ou de brome ou de radicaux méthyles, $X_2$ représente un atome d'hydrogène, de chlore ou de brome ou un radical alkyle ou alcoxy contenant de 1 à 4 atomes de carbone, et V représente O, S ou NH.

5. Procédé selon la revendication 1, caractérisé en ce qu'on part d'une iso-indolinone de formule 3), dans laquelle Y' représente un radical répondant à la formule:

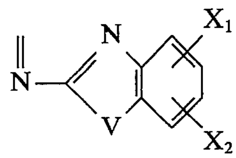

(12)

dans laquelle $X_1$ représente un atome d'hydrogène, de chlore ou de brome, un radical nitro, trifluorométhyle, carbamoyle ou sulfamoyle, un radical alkyle, alcoxy ou alkylsulfamoyle contenant de 1 à 4 atomes de carbone, un radical alcanoylamino, alkylcarbamoyle ou alcoxycarbonyle contenant de 2 à 6 atomes de carbone, ou un radical phénoxy, benzoylamino, phénylcarbamoyle, phénylsulfamoyle ou phénylazo éventuellement porteur d'atomes de chlore ou de brome ou de radicaux méthyles, $X_2$ représente un atome d'hydrogène, de chlore ou de brome ou un radical alkyle ou alcoxy contenant de 1 à 4 atomes de carbone, et V représente O, S ou NH.

6. Procédé selon la revendication 1, caractérisé en ce qu'on utilise, comme agent apte à céder un métal, un sel de nickel.

7. Procédé selon la revendication 1, caractérisé en ce qu'on utilise, comme solvant polaire, le diméthylformamide ou la N-méthylpyrrolidone.

8. Procédé selon la revendication 1, caractérisé en ce qu'on effectue la réaction à des températures comprises entre 100 et 200° C.